# EUROPÄISCHE PATENTSCHRIFT

(11) **EP 3 744 278 B1**
(45) Veröffentlichungstag und Bekanntmachung des Hinweises auf die Patenterteilung: **14.08.2024**
(21) Anmeldenummer: 19176751.6
(22) Anmeldetag: 27.05.2019
(51) Int. Cl.: A61B 18/14

(54) **ELEKTROCHIRURGISCHES INSTRUMENT UND VERFAHREN ZU DESSEN HERSTELLUNG**
ELECTRIC SURGICAL INSTRUMENT AND METHOD FOR ITS MANUFACTURE
INSTRUMENT ÉLECTROCHIRURGICAL ET SON PROCÉDÉ DE FABRICATION

(43) Veröffentlichungstag der Anmeldung: 02.12.2020
(73) Patentinhaber: Erbe Elektromedizin GmbH, 72072 Tübingen (DE)
(72) Erfinder: Felix, Bob, 72108 Rottenburg (DE)
(74) Vertreter: Rüger Abel Patentanwälte PartGmbB

(56) Entgegenhaltungen:
- EP-A1- 1 153 578
- EP-A2- 0 624 348
- EP-A2- 2 687 177
- US-A1- 2011 092 970

## Beschreibung

Die Erfindung betrifft ein elektrochirurgisches Instrument, das beispielsweise zur Thermofusion oder Koagulation verwendet werden kann, sowie ein Verfahren zu dessen Herstellung. Das elektrochirurgische Instrument hat zwei Branchen mit jeweils einem Maulteil, wobei jedes Maulteil eine Gewebekontaktfläche aufweist. Über die Gewebekontaktflächen kann ein Strom durch das dazwischen geklemmte Gewebe geleitet werden, um das Gewebe beispielsweise zu versiegeln.

Solche elektrochirurgischen Instrumente sind bekannt. Zum Beispiel beschreibt EP 1 372 512 B1 allgemein ein solches Instrument mit einem isolierenden und gegossenen Scharnier zur gelenkigen Lagerung der beiden Branchen aneinander.

EP 2 436 328 B1 und AU 2014 205 809 B2 offenbaren ein Verfahren und ein Instrument zum Verlegen von elektrischen Leitern durch ein bipolares elektrochirurgisches Instrument. Der Schwenkzapfen ist dabei zweiteilig mit einem Zwischenraum ausgebildet, so dass zumindest ein elektrischer Leiter durch den Zwischenraum des Schwenkzapfens hindurchgeführt werden kann.

EP 1 595 509 B1 betrifft ein elektrochirurgisches Instrument mit einem Isolationsteil, wobei ein Schwenkzapfen durch eine elektrisch isolierende Hülse des Isolationsteils hindurchgeführt ist. Um diese Hülse des Isolationsteils ist eine Vertiefung ausgebildet, in der ein gebogenes Ende einer Leitung angeordnet ist. Dieses Leitungsende ist über einen Kopplungsring axial mit dem Maulteil und der Gewebekontaktfläche einer Branche elektrisch kontaktiert.

US 8,771,270 B2 betrifft ein elektrochirurgisches Instrument mit zwei beweglich gelagerten Maulteilen. Die Maulteile sind mit einem Lagerteil um einen Schwenkzapfen herum schwenkbar gelagert. Das Lagerteil hat eine sich in Umfangsrichtung um den Schwenkzapfen erstreckende kreisbogenförmige Kontaktfläche, an der jeweils ein Federkontakt anliegt. Auf diese Weise können die Maulteile bzw. die Gewebekontaktflächen elektrisch kontaktiert werden.

EP 1 153 578 A1 beschreibt ein scheren- oder zangenförmiges chirurgisches Instrument mit zwei schwenkbar aneinander gelagerten Branchen. Die Branchen sind elektrisch leitfähig. Die Lagerwelle besteht aus Metall und verbindet einen Leiter in der einen Branche elektrisch leitend mit der zweiten Branche.

US 2011/0092970 A1 beschreibt ein elektrochirurgisches Instrument mit zwei schwenkbar aneinander gelagerten Branchen. Die Stromversorgung wird abhängig von der Schwenkstellung der Branchen relativ zueinander zugelassen oder unterbunden. Hierfür ist nur ein Teil des Schwenkzapfens sowie ein Teil der um den Schwenkzapfen schwenkbar gelagerten Branche elektrisch leitfähig. Abhängig von der Schwenkstellung um den Schwenkzapfen wird die elektrisch leitende Verbindung hergestellt oder unterbunden.

Bei elektrochirurgischen Instrumenten ist es gewünscht, die elektrischen Anschlüsse für die beiden Gewebekontaktflächen nur an einer Stelle, insbesondere an einer der Branchen, vorzusehen. Es ist bei vielen Instrumenten erforderlich eine elektrische Verbindung von einem der Leiter in der einen Branche mit der daran angeordneten schwenkbaren anderen Branche herzustellen. Eine solche elektrische Verbindung muss auch Ströme von mehr als 1 Ampere und bis zu 5 oder 6 Ampere sicher führen können. Die elektrische Verbindung darf die Handhabbarkeit des elektrochirurgischen Instruments und die Schwenklagerung nicht beeinträchtigen. Außerdem muss die elektrische Verbindung handhabungssicher ausgeführt und bei Mehrweginstrumenten leicht zu reinigen sein.

Es kann daher als Aufgabe der vorliegenden Erfindung angesehen werden, ein elektrochirurgisches Instrument zu schaffen, das den vorstehend beschriebenen Anforderungen genügt und sich einfach und kostengünstig herstellen lässt.

Diese Aufgabe wird durch ein elektrochirurgisches Instrument mit den Merkmalen des Patentanspruches 1 und ein Verfahren zu dessen Herstellung mit den Merkmalen des Patentanspruches 15 gelöst.

Das erfindungsgemäße elektrochirurgische Instrument hat eine erste Branche und eine zweite Branche. An der zweiten Branche ist ein eine Schwenkachse definierender Lagerzapfen angeordnet. An diesem Lagerzapfen ist die erste Branche schwenkbar gelagert. Jede Branche hat ein Maulteil im distalen Bereich ausgehend von der Schwenkachse betrachtet und ein Bedienteil ausgehend von der Schwenkachse betrachtet bis zum proximalen Ende des elektrochirurgischen Instruments. Beispielsweise ist an der ersten Branche oder einer einzigen anderen Stelle eine elektrische Verbindungseinrichtung für ein externes Kabel angeordnet. Die Verbindungseinrichtung ist über einen ersten Leiter elektrisch mit einer ersten Gewebekontaktfläche der ersten Branche und über einen zweiten Leiter elektrisch mit einer zweiten Gewebekontaktfläche der zweiten Branche verbunden. Die beiden elektrischen Leiter und die beiden Gewebekontaktflächen sind elektrisch zueinander isoliert, so dass an den beiden Gewebekontaktflächen unterschiedliche elektrische Potentiale anliegen können.

Jede Branche kann beispielsweise einen elektrisch leitfähigen Kern aufweisen, der zumindest teilweise mit einem elektrisch isolierenden Kunststoffmaterial umschlossen ist. Das elektrisch isolierende Kunststoffmaterial kann dabei eine elektrisch isolierende Außenschicht bilden und beispielsweise durch ein Spritzgussverfahren auf den Kern aufgebracht werden.

Der zweite Leiter ist integral ausgebildet und hat an seinem distalen Ende einen Kontaktabschnitt mit wenigstens einem Kontaktteil. Der wenigstens eine Kontaktteil liegt an einer Außenfläche, beispielsweise an einer radial nach außen ausgerichteten Umfangsfläche oder an einer axial ausgerichteten Ringfläche, eines elektrisch leitfähigen Bereichs des Lagerzapfens an. Insbesondere ist der wenigstens eine Kontaktteil erfindungsgemäß radial gegen die Umfangsfläche des Lagerzapfens vorgespannt. Dadurch ist eine elektrisch leitende Drehverbindung zwischen dem Lagerzapfen und dem wenigstens einen Kontaktteil hergestellt. Auf diese Weise kann eine elektrische Verbindung zwischen dem zweiten Leiter in der ersten Branche und der zweiten Gewebekontaktfläche in der zweiten Branche hergestellt werden. Der Lagerzapfen bildet somit einen Teil der elektrischen Verbindung zwischen dem zweiten Leiter und der zweiten Gewebekontaktfläche. In der zweiten Branche kann der Lagerzapfen beispielsweise über einen leitfähigen Kern der zweiten Branche mit der zweiten Gewebekontaktfläche verbunden sein. Die Verbindung zwischen dem Lagerzapfen und der zweiten Gewebekontaktfläche in der zweiten Branche kann auch mittels eines weiteren elektrischen Leiters oder auf sonstige Weise realisiert werden.

Die radiale Schleifkontaktierung zwischen dem wenigstens einen Kontaktteil und dem Lagerzapfen stellt eine sichere elektrische Verbindung her und lässt sich dabei sehr einfach herstellen. Der zweite Leiter einschließlich des Kontaktabschnitts kann sehr einfach durch Heraustrennen des zweiten Leiters mit dem Kontaktabschnitt und mit dem wenigstens einen Kontaktteil aus einer Platte hergestellt werden, beispielsweise durch Stanzen, durch Heraustrennen mittels eines Lasers, durch Wasserstrahlschneiden oder durch ein Ätzverfahren. Der zweite Leiter muss nach dem Heraustrennen aus der Platte nicht weiter bearbeitet werden, kann aber optional auch bearbeitet werden. Vorzugsweise erstreckt sich der zweite Leiter innerhalb der ersten Branche vollständig entlang bzw. in einer Erstreckungsebene.

Die beiden Leiter können zumindest abschnittsweise die Form bzw. Gestalt einer Leiterbahn haben. Der erste Leiter kann entsprechend dem zweiten Leiter integral ausgebildet sein. Er kann sich auch vollständig entlang bzw. in einer Erstreckungsebene erstrecken.

Es ist vorteilhaft, wenn der erste Leiter und der zweite Leiter in einer gemeinsamen Aussparung in der ersten Branche angeordnet sind. Alternativ dazu könnten die beiden Leiter auch in jeweils voneinander getrennten Aussparungen angeordnet sein. Die beiden Leiter können beispielsweise gemeinsam mit einem elektrisch leitfähigen Kern zur Herstellung der zweiten Branche in eine Form eingelegt und mit Kunststoff umspritzt bzw. in Kunststoff eingegossen werden, beispielsweise durch ein Spritzgussverfahren.

Es ist bevorzugt, wenn zwischen dem ersten Leiter und dem zweiten Leiter eine elektrisch isolierende Zwischenschicht angeordnet ist. Die Zwischenschicht ist dabei sozusagen sandwichartig zwischen den beiden Leitern positioniert. in Die elektrisch isolierende Zwischenschicht kann beispielsweise durch Anformen bzw. Aufspritzen auf den ersten Leiter und/oder zweiten Leiter hergestellt werden, bevor die beiden Leiter anschließend gemeinsam zur Bildung der zweiten Branche miteinander verbunden werden.

Es ist weiterhin vorteilhaft, wenn zwischen dem zweiten Leiter und einem elektrisch leitfähigen Kern der ersten Branche eine elektrisch isolierende Zwischenschicht angeordnet ist. Diese Zwischenschicht kann auch gleichzeitig die elektrisch isolierende Zwischenschicht zwischen dem zweiten Leiter und dem ersten Leiter bilden.

Es ist bevorzugt, wenn die elektrisch isolierende Zwischenschicht fest auf den zweiten Leiter aufgebracht ist, beispielsweise durch ein Spritzverfahren. Die elektrisch isolierende Zwischenschicht kann auch durch Kleben oder ein anderes Verfahren mit dem zweiten Leiter verbunden werden. Die Verbindung zwischen dem zweiten Leiter, der daran angebrachten Zwischenschicht und dem ersten Leiter findet vorzugsweise erst bei der Herstellung der ersten Branche statt.

Bei einem Ausführungsbeispiel hat der zweite Leiter einen sich an den Kontaktabschnitt anschließenden Leiterabschnitt mit einem rechteckförmigen Querschnitt. Bei einem bevorzugten Ausführungsbeispiel ist der Querschnitt im Hinblick auf seine Größe und/oder Form konstant.

Es ist außerdem vorteilhaft, wenn der zweite Leiter einen sich an den Leiterabschnitt anschließenden Anschlussabschnitt aufweist, der elektrisch mit der Verbindungseinrichtung verbunden ist oder einen Teil der Verbindungseinrichtung bildet. Bei einem Ausführungsbeispiel kann der Anschlussabschnitt eine plattenförmige Erweiterung des Leiterabschnitts sein.

Vorzugsweise hat der erste und/oder der zweite Leiter an jeder Stelle einen rechteckförmigen Querschnitt.

Bei einem Ausführungsbeispiel umgibt der Kontaktabschnitt den Lagerzapfen in einer Umfangsrichtung um die Schwenkachse vollständig. Bei einem anderen Ausführungsbeispiel kann der Kontaktabschnitt den Lagerzapfen in Umfangsrichtung nur teilweise umfassen, beispielsweise mindestens um 50% oder mindestens um 60% oder mindestens um 70% des Umfangs.

An dem Kontaktabschnitt ist wenigstens ein zum Lagerzapfen elastisch vorgespannter Halteteil ausgebildet, wobei an jedem Halteteil ein Kontaktteil oder mehrere Kontaktteile oder alle Kontaktteile angeordnet sind. Durch den integral mit dem zweiten Leiter ausgebildeten Halteteil kann auf zusätzliche Vorspannmittel verzichtet werden, um den wenigstens einen Kontaktteil gegen die Umfangsfläche des Lagerzapfens vorzuspannen.

Der Kontaktabschnitt kann außerdem wenigstens einen Stützteil aufweisen. Mittels des Stützteils kann sich der Kontaktabschnitt an einer dem Lagerzapfen radial mit Abstand gegenüberliegenden radial äußeren Seite an der ersten Branche abstützen. Beispielsweise kann sich der Stützteil über eine elektrisch isolierende Zwischenschicht an einem elektrisch leitfähigen Kern der zweiten Branche abstützen.

Es ist vorteilhaft, wenn zwischen dem wenigstens einen Stützteil und dem wenigstens einen Kontaktteil einen den Kontaktabschnitt vollständig durchsetzende Aussparung vorhanden ist. Zwischen dem Stützteil und dem Kontaktteil wird dadurch ein Freiraum geschaffen, um den Kontaktteil elastisch federnd gegen den Lagerzapfen vorzuspannen. Die Aussparung kann durch den Stützteil und/oder den Halteteil des Kontaktabschnitts umschlossen sein oder an einer Stelle offen sein.

Bei einem Ausführungsbeispiel hat der Lagerzapfen eine zylindrische Umfangsfläche. Zumindest der elektrisch leitfähige Bereich des Lagerzapfens ist durch eine Zylindermantelfläche gebildet. Bei einem anderen Ausführungsbeispiel kann der Lagerzapfen bzw. der elektrisch leitfähige Bereich des Lagerzapfens durch eine Mantelfläche eines Zylinderstumpfs gebildet sein. Der Lagerzapfen kann somit wenigstens einen konischen Abschnitt aufweisen oder insgesamt konisch ausgeführt sein.

Weist der Lagerzapfen wenigstens einen konischen Abschnitt auf, kann ein Drückteil vorhanden sein, das eine Axialkraft parallel zur Schwenkachse auf den wenigstens einen Kontaktteil erzeugt. Aufgrund der schräg zur Schwenkachse angeordneten elektrisch leitfähigen Fläche des Lagerzapfens wird dadurch auch eine radiale Andrückkraft zwischen dem wenigstens einen Kontaktteil und dem Lagerzapfen erzeugt.

Das vorstehend beschriebene elektrochirurgische Instrument lässt sich mit folgenden Schritten herstellen:
- Heraustrennen eines ersten Leiters aus einer Platte und eines zweiten Leiters aus einer Platte;
- elektrisches Verbinden des ersten Leiters mit einer ersten Gewebekontaktfläche;
- Anordnen der ersten Gewebekontaktfläche, des ersten Leiters und des zweiten Leiters in einer ersten Form und Herstellen einer ersten Branche durch Einfüllen eines fließfähigen, aushärtbaren Materials in die erste Form, derart, dass wenigstens ein Kontaktteil eines Kontaktbereichs des zweiten Leiters in einer Lageraussparung der ersten Brache angeordnet ist;
- Anordnen eines elektrisch leitfähiger Lagerzapfen und einer elektrisch mit dem Kern verbundenen zweiten Gewebekontaktfläche in einer zweiten Form und Herstellen einer zweite Branche durch Einfüllen eines fließfähigen, aushärtbaren Materials in die zweite Form; und
- Einstecken des Lagerzapfens der zweiten Branche in die Lageraussparung der ersten Branche zur gelenkigen Verbindung der beiden Branchen aneinander.

Ein anderer Aspekt des elektrochirurgischen Instruments betrifft die Ausgestaltung des Schwenkgelenks und die Führung eines optional vorhandenen Messers in einer Messerführungsaussparung. Diese Ausgestaltung des elektrochirurgischen Instruments kann unabhängig von den vorstehend beschriebenen Merkmalen der Stromzuführung realisiert werden.

Bei diesem weiteren erfinderischen Aspekt des elektrochirurgischen Instruments ist wiederum an der zweiten Branche der die Schwenkachse definierende Lagerzapfen vorhanden. In der ersten Branche ist eine Lageraussparung vorhanden, in die der Lagerzapfen hineinragt und das Schwenkgelenk bildet. Dadurch sind die beiden Branchen schwenkbar aneinander gelagert.

In der zweiten Branche ist eine Messerführungsaussparung vorhanden, in der ein Messer in einer Bewegungsrichtung bewegbar geführt gelagert ist. Die Messerführungsaussparung erstreckt sich entlang der Bewegungsrichtung an der Schwenkachse bzw. dem Lagerzapfen vorbei bis in das zweite Maulteil in den distalen Endbereich des elektrochirurgischen Instruments hinein. Der Lagerzapfen ist dabei in einer Querrichtung parallel zur Schwenkachse auf der einen Seite benachbart zur Messerführungsaussparung angeordnet. Auf der anderen entgegengesetzten Seite der Messerführungsaussparung ist die zweite Branche ohne Lagerzapfen und ohne Lageraussparung ausgebildet und weist keine Schwenklagerteile auf. Das Schwenkgelenk ist somit lediglich auf einer Seite bezüglich der Messerführungsaussparung vorhanden, während die jeweils andere Seite ohne Schwenkgelenk ausgebildet ist. Die Messerführungsaussparung erstreckt sich daher am Schwenkgelenk vorbei und muss nicht durch Lagerbestandteile des Schwenkgelenks hindurchgeführt werden.

Durch diese Ausgestaltung kann das Messer in Bewegungsrichtung in Höhe der Schwenkachse angeordnet werden und muss nicht exzentrisch zur Schwenkachse geführt gelagert werden. Die Schwenkachse durchsetzt sozusagen die Messerführungsaussparung. Dennoch kann das Schwenkgelenk sehr einfach ausgestaltet werden. Es sind keine Gelenkkonstruktionen notwendig, bei denen das Messer durch den Lagerzapfen hindurchgeführt werden muss. Auch das Anordnen von zwei separaten Schwenkgelenken entlang der Schwenkachse kann entfallen.

Somit ist eine einfache Herstellung und eine gute Zugänglichkeit zum Schwenkgelenk und zu der Messerführungsaussparung gewährleistet, um das elektrische Instrument nach dem Gebrauch reinigen zu können.

Vorzugsweise ist nur ein einziger Lagerzapfen und eine einzige Lageraussparung vorhanden, die ein einziges Schwenkgelenk und somit eine einzige Lagerstelle bilden.

Bei einem Ausführungsbeispiel weist die erste Branche eine erste Anlagefläche auf, in die die Lageraussparung mündet. Die zweite Branche kann eine zweite Anlagefläche aufweisen, von der der Lagerzapfen weg ragt. Die beiden Anlageflächen sind einander zugewandt und liegen insbesondere aneinander an.

Es ist außerdem vorteilhaft, wenn die erste Anlagefläche und/oder die zweite Anlagefläche eine Trennfläche bildet. Die beiden Branchen sind dabei nur über das Schwenkgelenk gelenkig aneinander gelagert, das sich in Querrichtung auf einer Seite der Trennfläche befindet. Die Trennfläche erstreckt sich vorzugsweise in einer Ebene.

Es ist vorteilhaft, wenn die Messerführungsaussparung zu der zweiten Anlagefläche offen ist. Dadurch wird das Reinigen der Messerführungsaussparung bzw. des Messers nach Gebrauch des Instruments vereinfacht. Bevorzugt ist die Messerführungsaussparung in Bewegungsrichtung des Messers betrachtet auf beiden Seiten der Schwenkachse bzw. des Lagerzapfens zu der zweiten Anlagefläche offen.

In der zweiten Branche kann bei einem bevorzugten Ausführungsbeispiel eine sich in Querrichtung erstreckende Queraussparung vorhanden sein. Die Queraussparung mündet an einem Ende in die Messerführungsaussparung und an ihrem in Querrichtung entgegengesetzten anderen Ende auf einer Außenseite der zweiten Branche aus. Diese Außenseite der zweiten Branche ist vorzugsweise von der ersten Branche abgewandt. Die Schwenkachse verläuft vorzugsweise durch die Queraussparung hindurch. Bei einem Ausführungsbeispiel kann die Queraussparung als Langloch ausgebildet sein. Das Langloch kann in Bewegungsrichtung des Messers eine Länge aufweisen, die mindestens so groß ist wie der Durchmesser des Lagerzapfens.

Mittels der Queraussparung ist die Zugänglichkeit der Messerführungsaussparung im Bereich des Lagerzapfens verbessert, beispielsweise zu Reinigungszwecken.

Der Lagerzapfen kann mit einer axialen Endfläche unmittelbar an die Messerführungsaussparung angrenzen. Der Lagerzapfen hat insbesondere keine Durchgangsöffnung, die zum Hindurchführen des Messers geeignet ist und die den Lagerzapfen zwischen seinen beiden axialen Endflächen durchsetzt. Der Lagerzapfen begrenzt die Messerführungsaussparung insbesondere an maximal drei Seiten.

Bei einer bevorzugten Ausführungsform ist in der ersten Branche eine Spülöffnung vorhanden. Die Spülöffnung mündet an einem Ende in die Lageraussparung und am entgegengesetzten anderen Ende an einer Außenseite der ersten Branche aus. Die Spülöffnung verläuft zwischen ihren beiden Enden in einer Erstreckungsrichtung im Wesentlichen rechtwinklig zur Schwenkachse. Die Spülöffnung ist vorzugsweise an einer der zweiten Branche zugewandten Seite offen. Die Spülöffnung mündet bei einem Ausführungsbeispiel an einer Schmalseite der ersten Branche.

Die Spülöffnung ist in einer vorgegebenen geöffneten Stellung der Branchen zwischen ihren beiden Mündungen entlang eines Abschnitts der Messeraussparung angeordnet. In dieser geöffneten Stellung erstreckt sich die Spülöffnung insbesondere vollständig entlang des Abschnitts der Messeraussparung. Durch die Spülöffnung ist der Zugang zur Messeraussparung weiter verbessert und das Reinigen des Instruments vereinfacht. Diese vorgegebene geöffnete Stellung ist beispielsweise die vollständige Öffnung der Branchen mit maximalem Öffnungswinkel zwischen den beiden Maulteilen der Branchen.

Bei sämtlichen Ausführungsbeispielen des elektrochirurgischen Instruments, die vorstehend erläutert wurden, kann das Instrument als Einweginstrument oder als Mehrweginstrument ausgeführt sein.

Vorteilhafte Ausgestaltungen der Erfindung ergeben sich aus den abhängigen Patentansprüchen, der Beschreibung und den Zeichnungen. Nachfolgend werden bevorzugte Ausführungsformen der Erfindung anhand der beigefügten Zeichnungen im Einzelnen erläutert. Es zeigen:
Figur 1 eine schematische Seitenansicht eines Ausführungsbeispiels eines elektrochirurgischen Instruments,
Figur 2 eine perspektivische Darstellung eines weiteren Ausführungsbeispiels eines elektrochirurgischen Instruments,
Figur 3 eine Seitenansicht eines Ausführungsbeispiels eines elektrochirurgischen Instruments sowie die elektrische Kontaktierung in einer blockschaltbildähnlichen Darstellung,
Figur 4 eine schematische Seitenansicht der ersten Branche und der zweiten Branche des elektrochirurgischen Instruments aus Figur 3,
Figur 5 einen distalen Endbereich eines elektrochirurgischen Instruments in einer perspektivischen Teildarstellung,
Figur 6 eine schematische Draufsicht auf einen distalen Endbereich eines elektrochirurgischen Instruments,
Figur 7 eine nicht erfindungsgemäße Querschnittsdarstellung durch ein Schwenkgelenk zweier schwenkbar aneinander gelagerter Branchen des elektrochirurgischen Instruments, wobei die Branchen in Explosionsdarstellung gezeigt sind,
Figur 8 einen Querschnitt durch den distalen Endbereich des elektrochirurgischen Instruments aus Figur 5 entlang der Schwenkachse S in einer perspektivischen Teildarstellung,
Figur 9 eine schematische perspektivische Teildarstellung der zwei Branchen des elektrochirurgischen Instruments in einer geöffneten Stellung der Branchen,
Figur 10 eine perspektivische Teildarstellung in einem Schnitt rechtwinklig zur Schwenkachse mit Blick auf einen Kontaktabschnitt eines zweiten elektrischen Leiters, der in der ersten Branche angeordnet ist,
Figur 11 eine Seitenansicht des zweiten elektrischen Leiters aus Figur 10,
Figuren 12 und 13 jeweils eine Prinzipskizze zur Veranschaulichung der elektrischen Verbindung zwischen dem Kontaktabschnitt des zweiten Leiters und einem elektrisch leitfähigen Umfangsbereich des Lagerzapfens,
Figur 14 eine schematische Darstellung eines weiteren Ausführungsbeispiels des Kontaktbereichs des zweiten Leiters,
Figuren 15-18 jeweils schematische Prinzipdarstellungen zur Ausgestaltung eines Kontaktteils des Kontaktbereichs des zweiten Leiters,
Figuren 20-23 jeweils eine schematische Querschnittsansicht durch ein nicht erfindungsgemäßes Ausführungsbeispiel eines elektrochirurgischen Instruments mit unterschiedlichen Ausgestaltungen der elektrischen Verbindung zwischen dem Kontaktabschnitt des zweiten Leiters in der ersten Branche und dem Lagerzapfen der zweiten Branche.

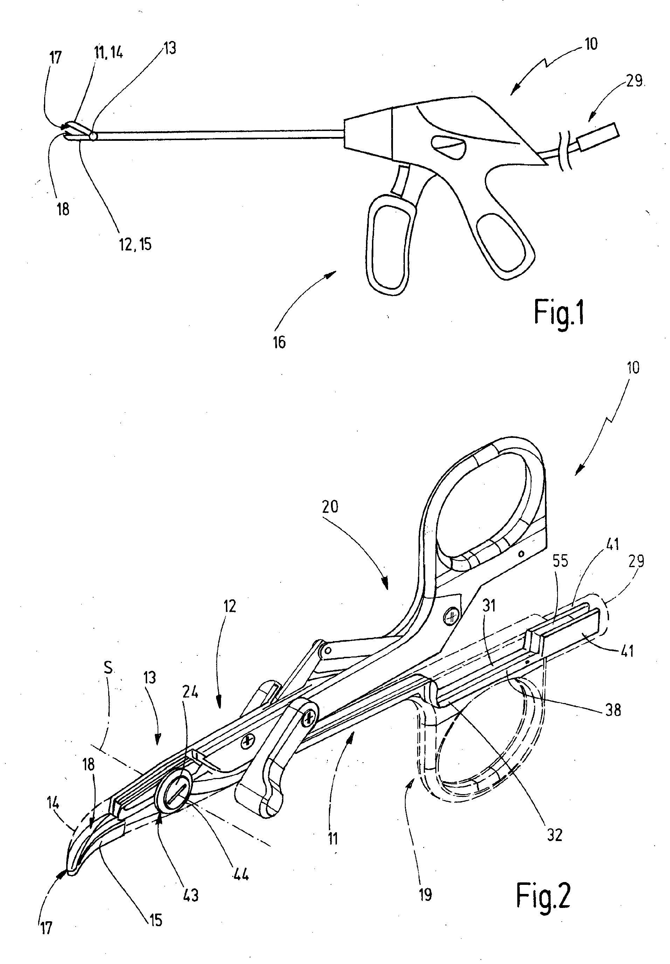

In den Figuren 1-4 ist jeweils ein Ausführungsbeispiel eines elektrochirurgischen Instruments 10 veranschaulicht, das beispielsgemäß als bipolares Instrument ausgebildet ist. Das elektrochirurgische Instrument hat eine erste Branche 11 und eine zweite Branche 12, die mittels eines Schwenkgelenks 13 um eine Schwenkachse S schwenkbar aneinander gelagert sind. Die Schwenkachse S erstreckt sich in einer Querrichtung Q.

Bei dem in Figur 1 dargestellten Ausführungsbeispiel sind von den Branchen 11, 12 im Wesentlichen die sich ausgehend von der Schwenkachse S bzw. dem Schwenkgelenk 13 in einem distalen Bereich erstreckenden Maulteile 14, 15 veranschaulicht. Das erste Maulteil 14 und/oder das zweite Maulteil 15 können schwenkbar gelagert sein. Zum Öffnen bzw. Schließen der Maulteile 14, 15 weist das Instrument 10 eine entsprechende Handhabungseinheit 16 auf. Mittels der Handhabungseinheit 16 können auch weitere Aktionen ausgeführt werden, beispielsweise das Anlegen einer Spannung zwischen einer ersten Gewebekontaktfläche 17 am ersten Maulteil 14 und einer zweiten Gewebekontaktfläche 18 am zweiten Maulteil 15 bzw. das Verursachen eines Stromflusses durch zwischen den Gewebekontaktflächen 17, 18 eingeklemmtem bzw. gehaltenem Gewebe. Zusätzlich oder alternativ kann auch ein Messer in einer Messerführungsaussparung entlang der Maulteile 14, 15 zum distalen Ende bewegt werden, beispielsweise um zwischen den Maulteilen 14, 15 eingeklemmtes bzw. gehaltenes Gewebe zu durchtrennen. Auf das Messer und die Messerführung wird nachfolgend näher eingegangen.

In den Figuren 2-4 sind weitere Ausführungsbeispiele eines elektrochirurgischen Instruments veranschaulicht, das jeweils scherenartig ausgebildet ist. Wie es in den Figuren zu erkennen ist, hat die erste Branche 11 ausgehend von der Schwenkachse S in Richtung zum distalen Ende das erste Maulteil 14 und ausgehend von der Schwenkachse S in Richtung zum proximalen Ende ein erstes Handhabungsteil 19. Entsprechend hierzu hat die zweite Branche 12 ausgehend von der Schwenkachse S in Richtung zum distalen Ende das zweite Maulteil 15 und ausgehend von der Schwenkachse S in Richtung zum proximalen Ende ein zweites Handhabungsteil 20.

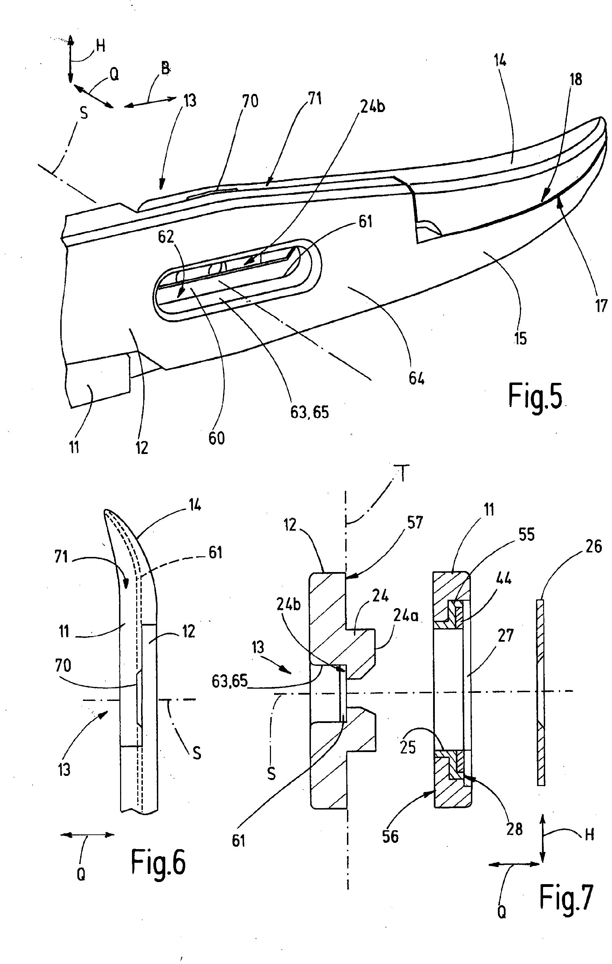

Zur Bildung des Schwenkgelenks 13 ist an der zweiten Branche 12 ein die Schwenkachse S definierender Lagerzapfen 24 vorhanden, der in eine an der ersten Branche 11 gebildete Lageraussparung 25 eingreift. Der Außendurchmesser des Lagerzapfens 24 entspricht - abgesehen von einem technisch erforderlichen Spiel - dem Innendurchmesser der Lageraussparung 25. Der Lagerzapfen 24 ragt in die Lageraussparung 25 hinein und bildet dadurch das Schwenkgelenk 13. Wie es beispielsweise schematisch in den Figuren 7-9 veranschaulicht ist, kann die Verbindung zwischen dem Lagerzapfen 24 und der Lageraussparung 25 mittels eines Sicherungsteils 26 gesichert werden, das mit einem freien Ende 24a des Lagerzapfens 24 verbunden werden kann. Das Sicherungsteil 26 kann eine plattenförmige Gestalt aufweisen und hat vorzugsweise eine kreisrunde Außenkontur.

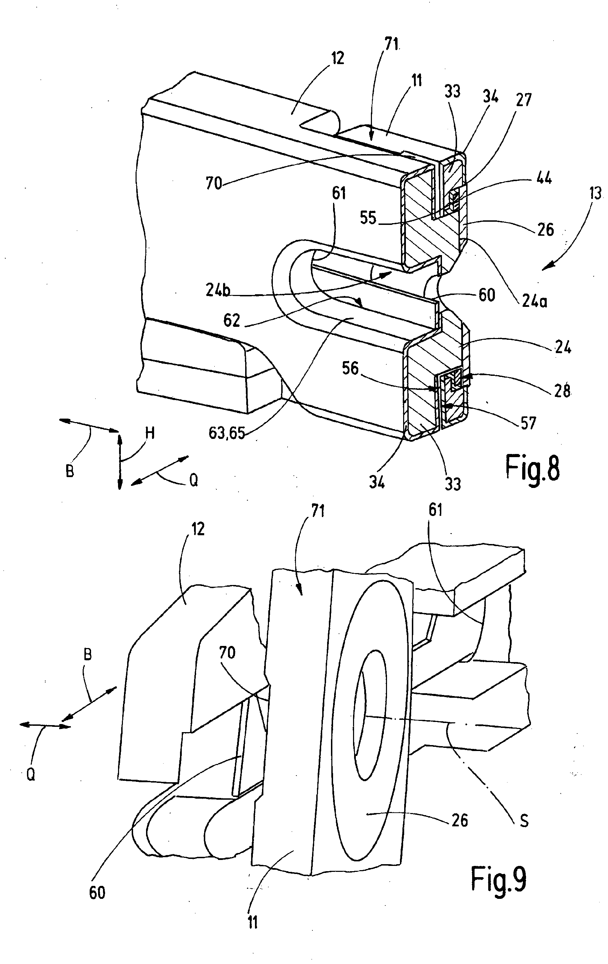

Wie es insbesondere in den Figuren 7 und 8 veranschaulicht ist, sitzt das Sicherungsteil 26 bei hergestellter Verbindung in einer an der ersten Branche 11 vorhanden Vertiefung 27, die die Lageraussparung 25 auf der der zweiten Branche 12 abgewandten Seite der ersten Branche 11 erweitert, so dass dadurch eine Ringschulter 28 im Übergang von der Lageraussparung 25 zu der Vertiefung 27 gebildet ist. An dieser Ringschulter 28 liegt das Sicherungsteil 26 bei hergestellter Verbindung an. Das Sicherungsteil 26 ist beispielsgemäß gleitbeweglich in der Vertiefung 27 relativ zu der ersten Branche 11 gelagert und drehfest mit der zweiten Branche 12 und beispielsgemäß dem Lagerzapfen 24 verbunden.

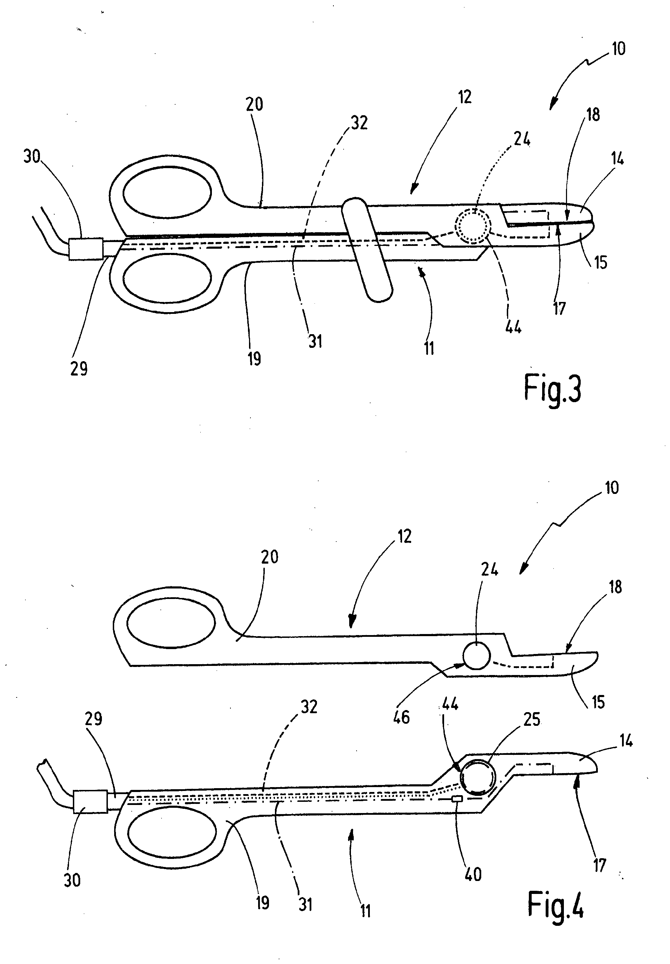

Zur elektrischen Kontaktierung weist das Instrument 10 eine elektrische Verbindungseinrichtung 29 auf, die dazu eingerichtet ist, mit einem externen elektrischen Anschluss 30 (Figuren 3 und 4) verbunden zu werden. Die elektrische Verbindung kann beispielsweise durch Ineinanderstecken der Verbindungseinrichtung 29 mit dem externen elektrischen Anschluss 30 hergestellt werden. Erforderlichenfalls können mechanische Sicherungsmittel vorhanden sein, um das versehentliche Auftrennen der elektrischen Verbindung zu verhindern.

Bei den in den Figuren 2-4 veranschaulichten Ausführungsbeispielen des elektrochirurgischen Instruments 10 ist die elektrische Verbindungseinrichtung 29 an einer der Branchen und beispielsgemäß der ersten Branche 11 vorhanden. Die elektrische Verbindungseinrichtung 29 befindet sich am proximalen Ende des ersten Handhabungsteils 19. Die elektrische Verbindungseinrichtung 29 ist zumindest zweipolig ausgeführt, wobei ein Pol mittels eines ersten Leiters 31 elektrisch mit der ersten Gewebekontaktfläche 17 und der zweite Pol mittels eines zweiten Leiters 32 elektrisch mit der zweiten Gewebekontaktfläche 18 verbunden ist. Die elektrischen Verbindungen sind insbesondere in den Figuren 3 und 4 veranschaulicht. Der erste Leiter 31 bildet zumindest einen Teil der elektrischen Verbindung zwischen der elektrische Verbindungseinrichtung 29 und der ersten Gewebekontaktfläche 17. Er kann sich zwar ausgehend von der Verbindungseinrichtung 29 bis zur ersten Gewebekontaktfläche 17 hin erstrecken, es können aber auch andere elektrisch leitfähige Bestandteile der ersten Branche 11 verwendet werden, um einen Teil der elektrischen Verbindung herzustellen, insbesondere im ersten Maulteil 14 der ersten Branche 11.

Der zweite Leiter 32 erstreckt sich ausgehend von der Verbindungseinrichtung 29 bis zum Schwenkgelenk 13. Durch das Schwenkgelenk 13 wird eine elektrische Verbindung zwischen dem zweiten Leiter 32 und der zweiten Branche 12 hergestellt. Die weitere elektrische Verbindung zwischen dem Schwenkgelenk 13 und der zweiten Gewebekontaktfläche 18 wird im Maulteil 15 der zweiten Branche 12 hergestellt.

Wie es stark schematisiert im perspektivischen Schnittbild der Figur 8 veranschaulicht ist, können die erste Branche 11 und die zweite Branche 12 zumindest im ersten Maulteil 14 und zweiten Maulteil 15 einen elektrisch leitfähigen Kern 33 aufweisen, der zumindest teilweise von einer elektrisch isolierenden Außenschicht 34 umschlossen ist. Die Außenschicht 34 ist in den Bereichen der jeweiligen Branche 11, 12 vorhanden, an denen keine elektrische Verbindung nach außen hin erfolgen soll. Bevorzugt ist die Außenschicht 34 außerhalb des Schwenkgelenks 13 und außerhalb der jeweiligen Gewebekontaktfläche 17, 18 überall vorhanden. Dadurch wird eine Berührsicherheit erreicht und ein Kurzschluss zwischen den elektrisch leitfähigen Bestandteilen der Branchen 11, 12 und insbesondere zwischen den jeweils elektrisch leitfähigen Kernen 33 verhindert.

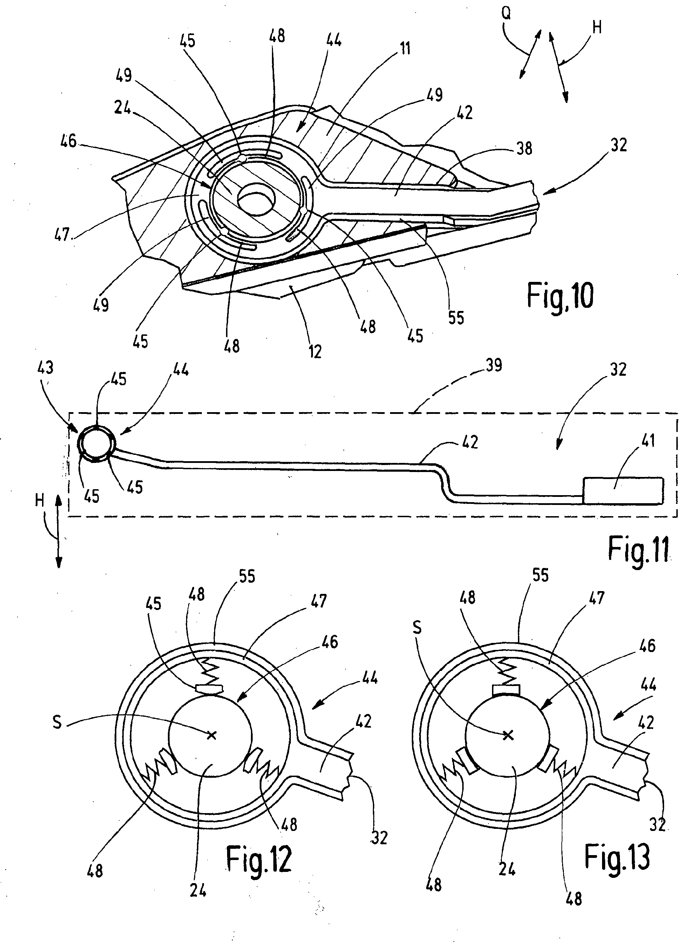

Wie es in den Figuren 2-4 veranschaulicht ist, sind die beiden Leiter 31, 32 in der ersten Branche 11 angeordnet. Sie können in einer gemeinsamen Aussparung in der ersten Branche 11 angeordnet sein (Figur 2). Die beiden Leiter 31, 32 sind beim Ausführungsbeispiel durch jeweils ein integrales Teil gebildet. Jeder Leiter 31 erstreckt sich entlang jeweils einer oder einer gemeinsamen Erstreckungsebene, die rechtwinklig zur Schwenkachse S ausgerichtet ist. Die Leiter 31, 32 können durch Heraustrennen aus einer Platte 39 hergestellt sein, wie es für den zweiten Leiter 32 schematisch in Figur 11 veranschaulicht ist. Das Heraustrennen kann durch Stanzen, Laserstrahlschneiden, Wasserstrahlschneiden, Ätzen, eine Kombination der vorgenannten Verfahren oder ein anderes Trennverfahren durchgeführt werden.

Der erste Leiter 31 und der zweite Leiter 32 sind in Querrichtung Q nebeneinander angeordnet und können zumindest abschnittsweise deckungsgleich verlaufen. Wie es in Figur 2 veranschaulicht ist, erstrecken sich die beiden Leiter 31, 32 durch das erste Handhabungsteil 19 überwiegend deckungsgleich, beispielsgemäß zumindest von der Verbindungseinrichtung entlang eines Bereichs des ersten Handhabungsteils 19, in dem das erste Handhabungsteil 19 und das zweite Handhabungsteil 20 rechtwinklig zur Querrichtung Q in einer Höhenrichtung H benachbart zueinander angeordnet sind. An diesen Bereich schließt sich ein Gelenkbereich mit dem Schwenkgelenk 13 an. Im Gelenkbereich liegen die beiden Branchen 11, 12 in Querrichtung Q nebeneinander. Im Gelenkbereich können die beiden Leiter 31, 32 zumindest noch abschnittsweise in Querrichtung Q betrachtet deckungsgleich verlaufen oder aber auch voneinander abweichende Verläufe aufweisen.

Wie es schematisiert in Figur 4 veranschaulicht ist, kann der erste Leiter 31 im Bereich der Lageraussparung 25 an einer Verbindungsstelle 40 mit dem Kern 33 elektrisch verbunden sein, beispielsweise durch Schweißen, Lötden oder Kleben. Zwischen der Verbindungsstelle 40 und der ersten Gewebekontaktfläche 17 wird die elektrische Verbindung durch den elektrisch leitfähigen Kern 33 der ersten Branche 11 bzw. des ersten Maulteils 14 hergestellt. Alternativ zu dieser Ausführungsform könnte sich der erste Leiter 31 auch durchgängig bis zur ersten Gewebekontaktfläche 17 erstrecken. Bei einer weiteren Ausführungsform könnte die Verbindungsstelle 40 im ersten Handhabungsteil 19 auch weiter entfernt von der Lageraussparung 25 angeordnet sein. Dies hängt davon ab, wie weit sich ein elektrisch leitfähiger Kern 33 ausgehend vom ersten Maulteil 14 innerhalb der ersten Branche 11 in Richtung zum proximalen Ende bzw. der elektrischen Verbindungseinrichtung 29 erstreckt.

Der erste Leiter 31 und der zweite Leiter 32 haben jeweils einen Anschlussabschnitt 41, der in etwa rechteckförmig konturiert sein kann. Alternativ dazu kann der Anschlussabschnitt 41 auch einen runden und insbesondere kreisrunden Querschnitt haben. Der Anschlussabschnitt 41 ist zumindest teilweise von außerhalb der ersten Branche 11 zugänglich und dient zur Herstellung der elektrischen Verbindung mit der elektrischen Verbindungseinrichtung 29. Die Anschlussabschnitte 41 können Bestandteile der elektrischen Verbindungseinrichtung 29 sein.

An den elektrischen Anschlussabschnitt 41 schließt sich sowohl beim ersten Leiter 31, als auch beim zweiten Leiter 32 ein Leiterabschnitt 42 an, der die Gestalt einer Leiterbahn aufweist. Der Leiterabschnitt 42 hat einen rechteckförmigen Querschnitt. In Querrichtung Q ist die Dicke des Leiterabschnitts 42 geringer als dessen Breite in Höhenrichtung H.

Wie bereits erläutert, kann der Leiterabschnitt 42 des ersten Leiters 31 an der Verbindungsstelle 40 elektrisch mit dem Kern 33 der ersten Branche 11 verbunden sein. Der Leiterabschnitt 42 des zweiten Leiters 32 hat an seinem dem Anschlussabschnitt 41 entgegengesetzten Ende, das als distales Ende 43 bezeichnet werden kann, einen Kontaktabschnitt 44 mit wenigstens einem und beispielsgemäß mehreren Kontaktteilen 45. Der Kontaktabschnitt 44 mit dem wenigstens einen Kontaktteil 45 ist dazu eingerichtet, eine elektrische Verbindung mit einem elektrisch leitfähigen Bereich des Lagerzapfens 24 herzustellen. Dazu liegt der wenigstens eine Kontaktteil 45 an einer Außenfläche und beispielsgemäß einer Umfangsfläche 46 des elektrisch leitfähigen Bereichs des Lagerzapfens 24 an. Beim Ausführungsbeispiel ist der Lagerzapfen 24 Bestandteil eines elektrisch leitfähigen Kerns 33 der zweiten Branche 12 und daher insgesamt elektrisch leitfähig. An seiner Umfangsfläche ist der Lagerzapfen 24 zumindest teilweise frei von der elektrisch isolierenden Außenschicht 34, so dass eine elektrische Verbindung mit dem wenigstens einen Kontaktteil 45 des Kontaktabschnitts 44 hergestellt werden kann.

In den Figuren 10 und 11 ist ein Ausführungsbeispiel eines Kontaktabschnitts 44 veranschaulicht. Bei diesem Ausführungsbeispiel umschließt der Kontaktabschnitt 44 den Lagerzapfen 24 in einer Umfangsrichtung um die Schwenkachse S vollständig. Die Kontaktteile 45 sind beispielsgemäß in Umfangsrichtung um die Schwenkachse S mit Abstand zueinander und insbesondere gleichmäßig verteilt angeordnet. Alternativ zu dieser Ausführungsform kann der Kontaktabschnitt 44 den Lagerzapfen 24 in Umfangsrichtung um die Schwenkachse S auch lediglich teilweise umschließen, vorzugsweise um mindestens 50% oder mindestens 60% oder mindestens 70%. Der Kontaktabschnitt 44 kann somit alternativ zu den veranschaulichten Ausführungsbeispielen auch gabelförmig ausgebildet sein.

In den Figuren 12 und 13 ist das Prinzip der elektrischen Kontaktierung zwischen der Umfangsfläche 46 des Lagerzapfens 24 und dem wenigstens einen Kontaktteil 45 veranschaulicht. Jeder Kontaktteil 45 kann parallel zur Schwenkachse S einen im Wesentlichen linienförmigen Kontakt mit der Umfangsfläche 46 des Lagerzapfens 24 aufweisen, wie es beispielhaft in Figur 12 gezeigt ist. Ein solch linienhafter Kontakt entsteht insbesondere dann, wenn der Kontaktteil 45 auf der der Umfangsfläche 46 zugewandten Seite eine Krümmung aufweist, die nicht mit der Krümmung der Umfangsfläche 46 übereinstimmt und beispielsweise konvex oder eben ausgebildet ist. Alternativ dazu kann jeder Kontaktteil 45 auf seiner der Umfangsfläche 46 zugewandten Seite eine konkave Krümmung aufweisen, die mit der Krümmung der Umfangsfläche 46 in Umfangsrichtung um die Schwenkachse S übereinstimmt, so dass ein im Wesentlichen flächiger Kontakt zwischen dem Kontaktteil 45 und der Umfangsfläche 46 entsteht (Figur 13).

Wie es in den Figuren 12 und 13 außerdem blockschaltbildähnlich, schematisch veranschaulicht ist, wird der wenigstens eine Kontaktteil 45 radial zur Schwenkachse S gegen die Umfangsfläche 46 des Lagerzapfens 24 gedrängt. Dies kann beispielsweise durch eine elastische Verformung zumindest eines Teils des Kontaktabschnitts 44 erreicht werden. Separate Vorspannmittel zum radialen Vorspannen des wenigstens einen Kontaktteils 45 gegen den Lagerzapfen 24 sind beispielsgemäß nicht vorhanden, sondern die radiale Vorspannkraft wird durch den Kontaktabschnitt 44 selbst erzeugt.

Hierfür weist der Kontaktabschnitt 44 wenigstens einen Stützteil 47 auf, der sich mittelbar oder unmittelbar an der ersten Branche 11 bzw. einer die Aussparung 38 begrenzenden Wand der ersten Branche 11 abstützt. Der Stützteil 47 kann, wie es in den Figuren 10 bis 13 veranschaulicht ist, ringförmig um die Schwenkachse S verlaufen.

Bei dem in den Figuren 10 und 11 veranschaulichten Ausführungsbeispiel weist der Kontaktabschnitt 44 außerdem wenigstens einen Halteteil 48 auf, an dem eines, mehrere oder alle der vorhandenen Kontaktteile 45 angeordnet sind. Der Halteteil 48 ist in Richtung zum Lagerzapfen 24 hin elastisch vorgespannt. Die Vorspannung wird durch eine elastische Verformung des Halteteils 48 erzeugt, wenn der wenigstens eine am Halteteil 48 angeordnete Kontaktteil 45 an der Umfangsfläche 46 des Lagerzapfens 24 anliegt und dadurch aus einer Ruhestellung - bei nicht elastisch verformtem Halteteil 48 - von der Schwenkachse S weg ausgelenkt wird. Durch diese Verformung des wenigstens einen Halteteils 48 wird erreicht, dass der daran angeordnete Kontaktteil 45 oder die daran angeordneten Kontaktteile 45 mit einer radialen Vorspannkraft gegen die Umfangsfläche 46 gedrückt werden.

In den Figuren 12 und 13 sind die Halteteile 48 schematisch als Federn dargestellt. Die konstruktive Ausgestaltung der Halteteile 48 kann variieren.

Bei dem in den Figuren 10 und 11 veranschaulichten Ausführungsbeispiel ist radial zur Schwenkachse S zwischen dem wenigstens einen Stützteil 47 und jedem Halteteil 48 eine den Kontaktabschnitt 44 in Querrichtung Q vollständig durchsetzende Durchbrechung 49 vorhanden. Die Durchbrechung 49 hat in Umfangsrichtung um die Schwenkachse S beispielsgemäß eine kreisbogenförmige Gestalt. Bei dem veranschaulichten Ausführungsbeispiel sind drei Halteteile 48 und drei daran angrenzende Durchbrechungen 49 vorhanden. Die Durchbrechungen 49 sind in Umfangsrichtung um die Schwenkachse S mit Abstand zueinander angeordnet. Die Halteteile 48 tragen beispielsgemäß in ihrem mittleren Bereich in Umfangsrichtung um die Schwenkachse S jeweils ein Kontaktteil 45. Der Kontaktteil 45 ist beispielsgemäß zylindrisch konturiert. Jeder Halteteil 48 bildet somit eine Art Blattfeder, wobei die jeweils benachbarte Durchbrechung 49 einen radialen Freiraum zum elastischen Verformen des jeweiligen Halteteils 48 ermöglicht.

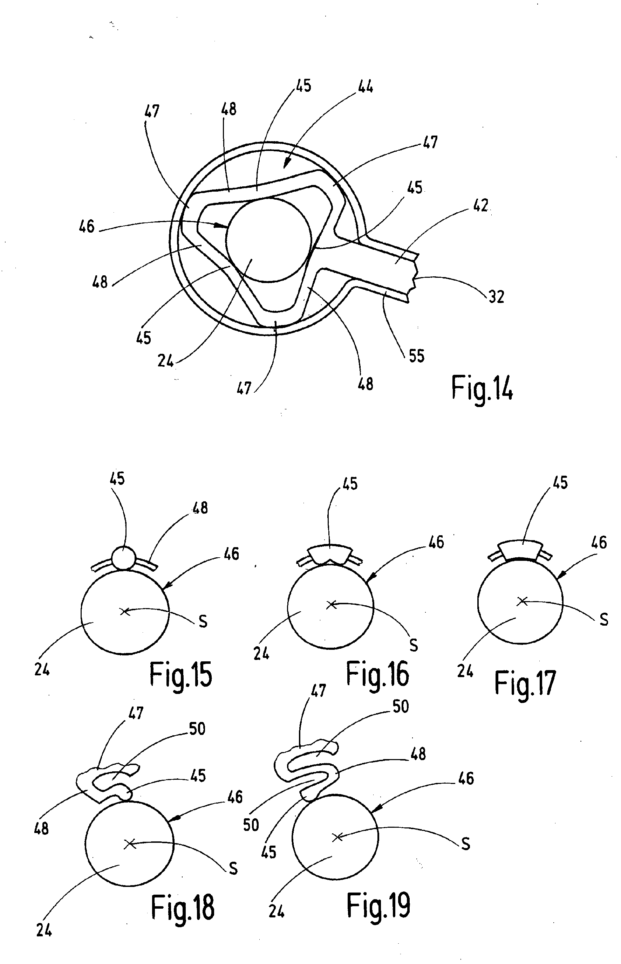

Anstelle eines zylindrisch konturierten Kontaktteils 45, wie es auch in der Prinzipdarstellung in Figur 15 veranschaulicht ist, kann an jedem Halteteil 48 auch wenigstens ein anders ausgebildeter Kontaktteil 45 angeordnet sein. In Figur 16 ist ein Ausführungsbeispiel für einen Kontaktteil 45 veranschaulicht, bei dem in Umfangsrichtung die Schwenkachse S zwei benachbart angeordnete konvex ballige Flächenabschnitte vorhanden sind, die an der Umfangsfläche 46 des Lagerzapfens 24 anliegen.

Das Ausführungsbeispiel des Kontaktteils 45 gemäß Figur 17 hat einen an der Umfangsfläche 46 des Lagerzapfens 24 anliegenden Flächenabschnitt, dessen Krümmung im Wesentlichen der Krümmung der Umfangsfläche 46 entspricht.

In den Figuren 18 und 19 sind alternative Ausgestaltungsmöglichkeiten für den Halteteil 48 dargestellt. Der Halteteil 48 ist bei diesen Ausführungsbeispielen einfach oder mehrfach gebogen und kann beispielsweise L-förmig (Figur 18) oder S-förmig (Figur 19) ausgebildet sein, so dass ein in Umfangsrichtung um die Schwenkachse S offener radialer Freiraum 50 zwischen dem wenigstens einen Kontaktteil 45 und dem Stützteil 47 bzw. mehrere solcher Freiräume 50 gebildet sind, so dass der wenigstens eine Kontaktteil 45 radial zur Schwenkachse S federelastisch bewegbar angeordnet ist.

Eine weitere Ausgestaltungsmöglichkeit für den Kontaktabschnitt 44 ist in Figur 14 gezeigt. Bei dieser Ausführung sind in Umfangsrichtung um die Schwenkachse S mehrere mit Abstand zueinander angeordnete Stützteile 47 angeordnet. Zwei unmittelbar benachbarte Stützteile 47 sind jeweils durch einen stegförmigen Halteteil 48 miteinander verbunden. Wenigstens ein Kontaktteil 45 ist am Halteteil 48 vorhanden bzw. durch den Halteteil 48 gebildet. Beim Ausführungsbeispiel sind die stegförmigen Halteteile 48 zur Schwenkachse S hin bogenförmig gekrümmt und liegen im jeweils mittleren Bereich zwischen zwei benachbarten Stützteilen 47 an der Umfangsfläche 46 des Lagerzapfens 24 an und bilden an dieser Stelle den Kontaktteil 45.

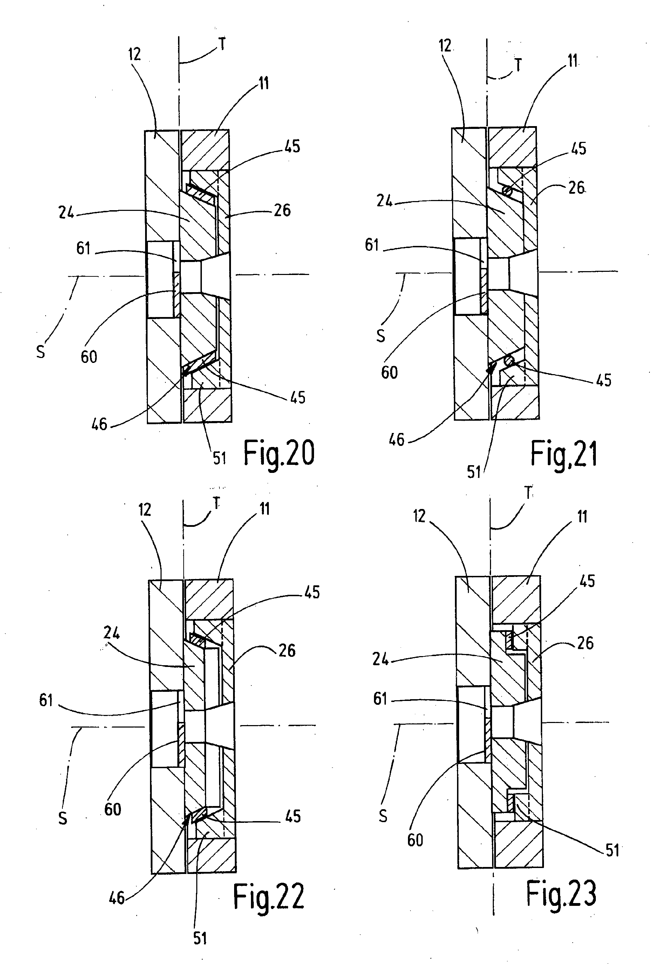

Bei den bisher beschriebenen Ausführungsbeispielen ist die Umfangsfläche 46 des Lagerzapfens 24 entsprechend einer Zylindermantelfläche koaxial zur Schwenkachse S ausgebildet. In den Figuren 20 bis 23 sind schematisch alternative, nicht erfindungsgemäße Ausgestaltungsmöglichkeiten für den Lagerzapfen 24 veranschaulicht, wobei insbesondere dessen Außenfläche zur Anlage mit dem Kontaktteil 45 verschiedene Konturen und/oder Ausrichtungen aufweist. Der Lagerzapfen 24 kann dabei insgesamt konisch sein (Figuren 20 und 21) oder zumindest einen konischen Abschnitt aufweisen (Figur 22). Die konische Form ist derart, dass sich der Lagerzapfen zumindest abschnittsweise zum Sicherungsteil 26 hin verjüngt. Der Bereich, an dem der wenigstens eine Kontaktteil 45 des Kontaktabschnitts 44 anliegt, ist bei diesen Ausführungsformen konisch ausgebildet. Der Kontaktteil 45 bzw. der Kontaktabschnitt 44 sind in den Figuren 20 bis 22 lediglich stark schematisiert dargestellt. Wie bei den bisherigen Ausführungsbeispielen können der Kontaktteil 45 bzw. der Kontaktabschnitt 44 unterschiedliche Ausgestaltungen annehmen.

Am Sicherungsteil 26 ist bei dieser Ausführungsform ein Drückteil 51 vorhanden, das den wenigstens einen Kontaktteil 45 axial in Richtung parallel zur Schwenkachse S gegen die Umfangsfläche 46 des konischen Abschnitts des Lagerzapfens 24 drückt. Das Drückteil 51 kann am Sicherungsteil 26 angeordnet sein, ein separates Teil bilden, das sich am Sicherungsteil 26 abstützt oder integral mit dem Sicherungsteil 26 ausgebildet sein. Durch die axiale Kraft auf den wenigstens einen Kontaktteil 45, entsteht aufgrund der schräg zur Schwenkachse S geneigt verlaufenden Umfangsfläche des Lagerzapfens 24 auch eine radiale Kraft, mit der der wenigstens eine Kontaktteil 45 gegen die Umfangsfläche 46 gedrängt wird. Bei dieser Ausführungsform kann auf einen in Radialrichtung elastisch federnd gelagerten Halteteil 48 im Kontaktabschnitt 44 verzichtet werden.

Bei der in Figur 23 veranschaulichten Ausführungsform weist die Außenfläche des Lagerzapfens 24 eine Ringfläche 52 auf, an der der wenigstens eine Kontaktteil 45 anliegen kann. Die Ringfläche 52 ist axial ausgerichtet und erstreckt sich beispielsgemäß in einer Ebene, die rechtwinkelig zur Schwenkachse S ausgerichtet ist. Beispielsweise kann die Ringfläche 52 durch eine Stufe des Lagerzapfens 24 gebildet sein, die eine Übergangsstelle zwischen aneinander angrenzenden Abschnitten des Lagerzapfens 24 mit unterschiedlichen Durchmessern darstellt. Der Abschnitt mit größerem Durchmesser des Lagerzapfens 24 schließt sich an die zweite Branche 12 an. Am Sicherungsteil 26 kann auch hier ein Drückteil 51 vorhanden sein, das den wenigstens einen Kontaktteil 45 axial in Richtung parallel zur Schwenkachse S gegen die Ringfläche 52 des Lagerzapfens 24 drückt.

Wie vorstehend erläutert, erstrecken sich der erste Leiter 31 und der zweite Leiter 32 in einer Aussparung 38 der zweiten Branche 11. Um eine elektrische Kontaktierung der beiden Leiter 31, 32 miteinander zu vermeiden, ist bei einem Ausführungsbeispiel eine elektrisch isolierende Zwischenschicht 55 zwischen den beiden Leitern 31, 32 angeordnet (Figur 2). Die elektrisch isolierende Zwischensicht 55 kann vorzugsweise fest mit einem der Leiter und beispielsgemäß mit dem zweiten Leiter 32 verbunden sei. Sie kann beispielsweise durch Herstellen einer formschlüssigen Verbindung und/oder einer Haftverbindung am zweiten Leiter 32 befestigt werden. Bei einem Ausführungsbeispiel kann die Zwischenschicht 55 an dem zweiten Leiter 32 angeformt bzw. durch ein Spritzgussverfahren mit dem zweiten Leiter 32 verbunden werden.

Die Zwischenschicht 55 am zweiten Leiter 32 dient beispielsgemäß auch dazu, eine elektrische Isolierung zwischen dem zweiten Leiter 32 und dem elektrisch leitfähigen Kern 33 der ersten Branche 11 sicherzustellen. Die Zwischenschicht 55 kann den zweiten Leiter 32 an einer oder an mehreren Seiten umschließen, wie es schematisch in Figur 10 veranschaulicht ist. Beispielsweise kann sich der wenigstens eine Stützteil 47 mittels der Zwischenschicht 55 in der Aussparung 38 an der ersten Branche 11 abstützen.

Das elektrochirurgische Instrument 10 gemäß einem der vorstehend beschriebenen Ausführungsbeispiele kann wie folgt hergestellt werden:
Die beiden Leiter 31, 32 werden aus einer Platte 39 oder aus verschiedenen Platten 39 herausgetrennt, beispielweise durch Stanzen, Laserstrahlschneiden, Wasserstrahlschneiden, Ätzen oder dergleichen. Bevorzugt wird dann auf den zweiten Leiter 32 eine elektrisch isolierende Zwischenschicht 55 aufgebracht, beispielweise in dem der zweite Leiter 32 in eine Form eingelegt und zumindest teilweise mit der elektrisch isolierenden Zwischenschicht 55 umspritzt wird.

Anschließend werden die beiden Leiter 31, 32, getrennt durch die Zwischenschicht 55 gemeinsam mit einer Elektrode, die die erste Gewebekontaktfläche aufweist, in eine erste Form eingelegt. Durch Einfüllen von fließfähigem, aushärtbaren Materialien die erst Form und anschließendes Aushärten wird die erste Branche 11 hergestellt, die dann die erste Gewebekontaktfläche 17 und die beiden Leiter 31, 32 aufweist. Die erste Branche 11 wird in der ersten Form derart hergestellt, dass eine Lageraussparung 25 gebildet ist.

Ein elektrisch leitfähiger Lagerzapfen 24 und eine elektrisch leitfähige Elektrode mit der zweiten Gewebekontaktfläche 18 wird in einer zweiten Form angeordnet, in die fließfähiges, aushärtbares Material eingefüllt wird und durch Aushärten wird die zweite Branche 12 hergestellt. Nach dem Herstellen der beiden Branchen 11, 12 werden diese durch Einstecken des Lagerzapfens 24 in die Lageraussparung 25 gelenkig miteinander verbunden. Bei der Herstellung der gelenkigen Verbindung gelangt der wenigstens eine Kontaktteil 45 des Kontaktbereichs 44 in Kontakt mit der Umfangsfläche des elektrisch leitfähigen Lagerzapfens 24, so dass ein elektrisch leitfähiger Kontakt des zweiten Leiters 32 mit dem Lagerzapfen 24 und über den Lagerzapfen 24 mit der zweiten Gewebekontaktfläche 18 hergestellt ist.

Bei dem bevorzugten Ausführungsbeispiel des elektrochirurgischen Instruments 10 ist lediglich ein einziges Schwenkgelenk 13 mit einem einzigen Lagerzapfen 24 und einer einzigen Lageraussparung 25 vorhanden, in die der Lagerzapfen 24 hineinragt.

Die Lageraussparung 25 mündet auf der dem Sicherungsteil 26 entgegengesetzten Seite in eine erste Anlagefläche 56. Die erste Anlagefläche 56 erstreckt sich in einer Ebene rechtwinklig zur Schwenkachse S. Die erste Anlagefläche 56 kann an der Außenseite eines Bereichs der elektrisch isolierenden Außenschicht 34 vorhanden sein, die den Kern 33 der ersten Branche 11 umschließt (Figur 8).

An der zweiten Branche 12 ist eine zweite Anlagefläche 57 vorhanden, ausgehend von der sich der Lagerzapfen 24 zu seinem freien Ende 24a hin erstreckt. Die zweite Anlagefläche 57 erstreckt sich vorzugsweise in einer Ebene rechtwinklig zur Schwenkachse S. Die zweite Anlagefläche 57 kann durch die Außenseite eines Abschnitts der elektrisch isolierenden Außenschicht 34 um den Kern 33 der zweiten Branche 12 gebildet sein (Figur 8).

Die erste Anlagefläche 56 und/oder die zweite Anlagefläche 57 bilden eine Trennfläche T und beispielsgemäß eine Trennebene (Figur 7). Die beiden Anlageflächen 56, 57 liegen aneinander an oder sind einander zugewandt und benachbart zueinander angeordnet. Vorzugsweise liegen die beiden Anlageflächen 56, 57 unmittelbar aneinander an. Bei einem abgewandelten Ausführungsbeispiel kann alternativ ein Gleitring oder dergleichen zwischen den beiden Anlageflächen 56, 57 angeordnet sein.

Das durch den Lagerzapfen 24 und die Lageraussparung 25 gebildete Schwenkgelenk 13 ist in Querrichtung Q betrachtet lediglich auf einer Seite der Trennfläche T bzw. Trennebene angeordnet. Auf der anderen Seite ist kein Schwenkgelenk oder keine Schwenklagerstelle vorhanden.

Die durch die zweite Anlagefläche 57 gebildete Trennfläche T wird im Umfangsbereich um das Schwenkgelenk 13 nicht von der ersten Branche 11 durchsetzt. Die Trennfläche T stellt in diesem Fall auch eine Begrenzungsebene im Gelenkbereich des Schwenkgelenks 13 dar, durch die die erste Branche 11 nicht hindurchragt.

Ein Ausführungsbeispiel des elektrochirurgischen Instruments 10 weist ein Messer 60 auf, das in einer Bewegungsrichtung B rechtwinklig zur Querrichtung Q geführt bewegbar gelagert ist. Die Bewegungsrichtung B erstreckt sich parallel zur Erstreckungsrichtung der zweiten Branche 12. Die Bewegungsrichtung B kann abhängig von der Ausgestaltung der Branchen insbesondere der Mauteile 14, 15 zumindest teilweise gekrümmt verlaufen.

Zur Führung des Messers 60 ist in der zweiten Branche 12 eine Messerführungsaussparung 61 vorhanden, die beispielsgemäß einen rechteckförmigen Querschnitt aufweist. Das Messer 60 kann sich seitlich an den Wänden der Messerführungsaussparung 61 abstützen kann. Die Messerführungsaussparung 61 hat einen Führungsabschnitt 62, der sich in Bewegungsrichtung B im Gelenkbereich des Schwenkgelenks 13 und mithin im Bereich des Lagerzapfens 24 erstreckt. Entlang dieses Führungsabschnitts 62 ist in der zweiten Branche 12 eine Queraussparung 63 vorhanden, die sich in Querrichtung Q erstreckt und von der Schwenkachse S durchsetzt wird. Die Queraussparung 63 mündet an einem Ende in den Führungsabschnitt 62 der Messerführungsaussparung 61 und mündet am in Querrichtung Q entgegengesetzten Ende in eine Außenseite 64 der zweiten Branche 12. Beispielsgemäß ist diese Außenseite 64 der ersten Branche 11 abgewandt.

Die Queraussparung 63 ist beim Ausführungsbeispiel als ein sich in Bewegungsrichtung B erstreckendes Langloch 65 ausgebildet. Die Länge der Queraussparung 63 bzw. des Langlochs 65 in Bewegungsrichtung B entlang des Führungsabschnitts 62 der Messerführungsaussparung 61 ist mindestens so groß und beispielsgemäß größer als der Außendurchmesser des Lagerzapfens 24. Über die Queraussparung 63 ist die Zugänglichkeit zur Messerführungsaussparung 61 gewährleistet, die in Querrichtung Q auf der anderen Seite zumindest teilweise durch eine axiale Endfläche 24b des Lagerzapfens 24 begrenzt wird. Die axiale Endfläche 24b schließt sich beim Ausführungsbeispiel unmittelbar an den Führungsabschnitt 62 der Messerführungsaussparung 61 an.

Der Lagerzapfen 24 erstreckt sich in Querrichtung Q ausgehend von der axialen Endfläche 24b bis zum freien Ende 24a. Rechtwinklig zur Schwenkachse S ist der Lagerzapfen 24 beispielsgemäß durchbrechungslos ausgeführt. Zumindest hat der Lagerzapfen 24 keine mit der Messerführungsaussparung 61 fluchtende Durchbrechung zwischen seiner axialen Endfläche 24b und seinem freien Ende 24a. Er ist vielmehr in Querrichtung Q versetzt zur Messerführungsaussparung 61 angeordnet.

Die Messerführungsaussparung 61 ist in Bewegungsrichtung B betrachtet auf beiden Seiten der axialen Endfläche 24b des Lagerzapfens 24 zur zweiten Anlagefläche 57 und mithin auch zur ersten Anlagefläche 56 hin offen. Bei geschlossenen Branchen 11, 12, wenn die Maulteile 14, 15 in Bereich der Gewebekontaktflächen 17, 18 aneinander anliegen, wird die Messerführungsaussparung 61 in Bewegungsrichtung B benachbart zur axialen Endfläche 24b des Lagerzapfens 24 durch die erste Anlagefläche 56 begrenzt.

Wie es insbesondere in Figur 7 zu erkennen ist, erstreckt sich die axiale Endfläche 24b des Lagerzapfens 24 beispielsgemäß in derselben Ebene wie die zweite Anlagefläche 57. Die axiale Endfläche 24b könnte auch versetzt zur zweiten Anlagefläche 57 angeordnet sein. Jedenfalls wird die Messerführungsaussparung 61 in dem Bereich, in dem sie an die axiale Endfläche 24b des Lagerzapfens 24 angrenzt, an maximal drei Seiten von dem Lagerzapfen 24 begrenzt.

Durch den Verlauf der Messerführungsaussparung 61 durch die Schwenkachse S hindurch ist eine exzentrische Anordnung des Messers 60 vermieden. Das Messer 60 macht dadurch auch bei einer Schwenkbewegung der Branchen 11, 12 relativ zueinander keine exzentrische Bewegung um die Schwenkachse S. Dadurch, dass das Schwenkgelenk 13 nur auf einer Seite in Bezug auf die Messerführungsaussparung 61 angeordnet ist, wird eine einfache und kostengünstig herstellbare Ausgestaltung des elektrochirurgischen Instruments 10 erreicht, die eine gute Zugänglichkeit zu den Aussparungen ermöglicht und daher auch eine vorteilhafte Reinigung bzw. Sterilisation gewährleistet.

Bei einem bevorzugten Ausführungsbeispiel ist in der ersten Branche 11 außerdem eine Spülöffnung 70 vorhanden, die sich im Wesentlichen rechtwinklig zur Schwenkachse S erstreckt. Die Spülöffnung 70 mündet an einem Ende in die Lageraussparung 25 und an ihrem entgegengesetzten Ende an einer Außenseite 71 der ersten Branche 11 aus. Die Spülöffnung 71 ist beim Ausführungsbeispiel in Höhenrichtung H versetzt zur Schwenkachse S angeordnet. Sie erstreckt sich in etwa radial zur Schwenkachse S.

Bei einer Schwenkbewegung der beiden Branchen 11, 12 relativ zueinander, führt die Spülöffnung 70 eine Schwenkbewegung um die Schwenkachse S aus. In einer vorgegebenen geöffneten Stellung, wenn die beiden Gewebekontaktflächen 17, 18 mit Abstand zueinander angeordnet sind, ist die Erstreckungsrichtung der Spülöffnung 70 parallel zur Bewegungsrichtung B des Messers 60 ausgerichtet und die Spülöffnung 70 erstreckt sich entlang eines Abschnitts der Messerführungsaussparung 61, wie es schematisiert in Figur 9 veranschaulicht ist. Dadurch ist der Zugang zur Messerführungsaussparung 61 und dem darin geführt angeordneten Messer 60 verbessert, insbesondere im Hinblick auf die Reinigung des Instruments 10 nach seiner Verwendung.

Die vorstehend beschriebenen Ausführungsbeispiele des elektrochirurgischen Instruments 10 können miteinander kombiniert oder unabhängig voneinander ausgebildet sein. Insbesondere ist die Anordnung der Messerführungsaussparung 61 unter Führung des Messers 60 seitlich versetzt zum Schwenkgelenk 13 unabhängig von der Ausgestaltung der elektrischen Kontaktierung der Gewebekontaktflächen 17,18 über die beiden Leiter 31, 32. Diese Aspekte können auch separat voneinander realisiert werden.

Die Erfindung betrifft ein elektrochirurgisches Instrument 10 mit einer ersten Branche 11 und einer zweiten Branche 12, die mittels eines Lagerzapfens 24 der zweiten Branche 12 schwenkbar aneinander gelagert sind. Die erste Branche 11 hat eine erste Gewebekontaktfläche 17 und die zweite Branche 12 hat eine zweite Gewebekontaktfläche 18. Die beiden Gewebekontaktflächen 17, 18 sind mit einer elektrischen Verbindungseinrichtung 29 an der ersten Branche 11 elektrisch verbunden. Die elektrische Verbindung wird zumindest teilweise über einen jeweiligen Leiter 31 bzw. 32 hergestellt, die an oder in der ersten Branche 11 verlaufen. Der zweite Leiter 32 und optional auch der erste Leiter 31 sind jeweils integral durch einen einzigen Körper gebildet, der beispielweise durch Heraustrennen aus einer Platte 39 hergestellt werden kann. Der zweite Leiter 32 hat einen Kontaktabschnitt 44 mit wenigstens einem Kontaktteil 45, das an einer elektrisch leitfähigen Umfangsfläche 46 des Lagerzapfens 24 anliegt, so dass eine elektrische Drehverbindung zwischen dem Kontaktabschnitt 44 und dem Lagerzapfen 24 hergestellt ist. Auf diese Weise kann der in der ersten Branche 11 angeordnete zweite Leiter 32 eine elektrische Verbindung über den Lagerzapfen 24 mit der zweiten Branche 12 und weiter mit der zweiten Gewebekontaktfläche 18 herstellen.

### Bezugszeichenliste:

- 10: elektrochirurgisches Instrument
- 11: erste Branche
- 12: zweite Branche
- 13: Schwenkgelenk
- 14: erstes Maulteil
- 15: zweites Maulteil
- 16: Handhabungseinheit
- 17: erste Gewebekontaktfläche
- 18: zweite Gewebekontaktfläche
- 19: erstes Handhabungsteil
- 20: zweites Handhabungsteil

- 24: Lagerzapfen
- 24a: freies Ende des Lagerzapfens
- 24b: axiale Endfläche des Lagerzapfens
- 25: Lageraussparung
- 26: Sicherungsteil
- 27: Vertiefung
- 28: Ringschulter
- 29: elektrische Verbindungseinrichtung
- 30: externer elektrischer Anschluss
- 31: erster Leiter
- 32: zweiter Leiter
- 33: Kern
- 34: Außenschicht

- 38: Aussparung
- 39: Platte
- 40: Verbindungsstelle
- 41: Anschlussabschnitt
- 42: Leiterabschnitt
- 43: distales Ende des zweiten Leiters
- 44: Kotaktabschnitt
- 45: Kontaktteil
- 46: Umfangsfläche
- 47: Stützteil
- 48: Halteiteil
- 49: Durchbrechung
- 50: Freiraum
- 51: Drückteil
- 52: Ringfläche

- 55: Zwischenschicht
- 56: erste Anlagefläche
- 57: zweite Anlagefläche

- 60: Messer
- 61: Messerführungsaussparung
- 62: Führungsabschnitt
- 63: Queraussparung
- 64: Außenseite der zweiten Branche
- 65: Langloch

- 70: Spülöffnung
- 71: Außenseite der ersten Branche

- B: Bewegungsrichtung
- Q: Querrichtung
- S: Schwenkachse
- T: Trennfläche

## Patentansprüche

1. Elektrochirurgisches Instrument (10)
mit einer ersten Branche (11) und einer zweiten Branche (12), die einen eine Schwenkachse (S) definierenden Lagerzapfen (24) aufweist, an dem die erste Branche (11) schwenkbar gelagert ist,
mit einer elektrischen Verbindungseinrichtung (29) für einen externen elektrischen Anschluss (30), wobei die elektrische Verbindungseinrichtung (29) über einen ersten Leiter (31) elektrisch mit einer ersten Gewebekontaktfläche (17) der ersten Branche (11) und über einen zweiten Leiter (32) elektrisch mit einer zweiten Gewebekontaktfläche (18) der zweiten Branche (12) verbunden ist,
wobei der zweite Leiter (32) integral ausgebildet ist und an seinem distalen Ende (43) einen Kontaktabschnitt (44) mit wenigstens einem Kontaktteil (45) aufweist, der an einer Außenfläche eines elektrisch leitfähigen Bereichs des Lagerzapfens (24) anliegt, so dass eine elektrische Drehverbindung hergestellt ist,
**dadurch gekennzeichnet, dass** der Kontaktabschnitt (44) wenigstens einen zum Lagerzapfen (24) hin elastisch vorgespannten Halteteil (48) aufweist, an dem jeweils ein Kontaktteil (45) oder mehrere Kontaktteile (45) von dem wenigstens einen Kontaktteil (45) angeordnet sind.

2. Elektrochirurgisches Instrument nach Anspruch 1, wobei die elektrischen Verbindungseinrichtung (29) an der ersten Branche (11) angeordnet ist.

3. Elektrochirurgisches Instrument nach Anspruch 1 oder 2, wobei eine elektrisch isolierende Zwischenschicht (55) zwischen dem ersten Leiter (31) und dem zweiten Leiter (32) angeordnet ist.

4. Elektrochirurgisches Instrument nach einem der vorhergehenden Ansprüche, wobei jede Branche (11, 12) einen elektrisch leitfähigen Kern (33) und eine den Kern (33) zumindest teilweise umgebende elektrisch isolierende Außenschicht (34) aufweist.

5. Elektrochirurgisches Instrument nach Anspruch 4, wobei eine elektrisch isolierende Zwischenschicht (55) zwischen dem zweiten Leiter (32) und dem Kern (33) der ersten Branche (11) angeordnet ist.

6. Elektrochirurgisches Instrument nach Anspruch 3 oder 5, wobei die elektrisch isolierende Zwischenschicht (55) durch eine fest auf den zweiten Leiter (32) aufgebrachte Schicht gebildet ist.

7. Elektrochirurgisches Instrument nach einem der vorhergehenden Ansprüche, wobei der zweite Leiter (32) einen sich an den Kontaktanschnitt (44) anschließenden Leiterabschnitt (42) mit einem rechteckförmigen Querschnitt aufweist.

8. Elektrochirurgisches Instrument nach Anspruch 7, wobei der zweite Leiter (32) einen sich an den Leiterabschnitt (42) anschließenden Anschlussabschnitt (41) aufweist, der elektrisch mit der Verbindungseinrichtung (29) verbunden ist.

9. Elektrochirurgisches Instrument nach einem der vorhergehenden Ansprüche, wobei der Kontaktabschnitt (44) den Lagerzapfen (24) in einer Umfangsrichtung um die Schwenkachse (S) vollständig umschließt.

10. Elektrochirurgisches Instrument nach einem der vorhergehenden Ansprüche, wobei der Kontaktabschnitt (44) wenigstens einen Stützteil (47) aufweist, mittels dem sich der Kontaktanschnitt (44) an einer dem Lagerzapfen (24) entgegengesetzten radial äußeren Seite an der ersten Branche (11) abstützt.

11. Elektrochirurgisches Instrument nach Anspruch 10, wobei zwischen dem wenigstens einen Stützteil (47) und dem wenigstens einen Kontaktteil (45) eine den Kontaktabschnitt (44) vollständig durchsetzende Durchbrechung (49) vorhanden ist.

12. Elektrochirurgisches Instrument nach einem der vorhergehenden Ansprüche, wobei Lagerzapfen (24) einen konischen Abschnitt aufweist, an dem der wenigstens eine Kontaktteil (45) des Kontaktabschnitts (44) anliegt.

13. Elektrochirurgisches Instrument nach Anspruch 12, wobei ein Drückteil (51) vorhanden ist, das eine Axialkraft parallel zur Schwenkachse (S) auf den wenigstens einen Kontaktteil (45) erzeugt.

14. Verfahren zur Herstellung eines elektrochirurgischen Instruments (10) nach einem der Ansprüche 1-13 mit folgenden Schritten:
- Heraustrennen eines ersten Leiters (31) aus einer Platte (39) und eines zweiten Leiters (32) aus einer Platte (39),
- elektrisches Verbinden des ersten Leiters (31) mit einer ersten Gewebekontaktfläche (17),
- Anordnen der ersten Gewebekontaktfläche (17), des ersten Leiters (31) und des zweiten Leiters (32) in einer ersten Form und Herstellen einer ersten Branche (11) durch Einfüllen eines fließfähigen, aushärtbaren Materials in die erste Form, derart, dass wenigstens ein Kontaktteil (45) eines Kontaktbereichs (44) des zweiten Leiters (32) in einer Lageraussparung (25) der ersten Brache (11) angeordnet ist,
- Anordnen eines elektrisch leitfähigen Lagerzapfens (24) und einer elektrisch mit dem Kern (33) verbundenen zweiten Gewebekontaktfläche (18) in einer zweiten Form und Herstellen einer zweite Branche (12) durch Einfüllen eines fließfähigen, aushärtbaren Materials in die zweite Form,
- Einstecken des Lagerzapfens (24) der zweiten Branche (12) in die Lageraussparung (25) der ersten Branche (11) zur gelenkigen Verbindung der beiden Branchen (11, 12) aneinander.

## Claims

1. Electrosurgical instrument (10)
having a first branch (11) and a second branch (12) that comprises a support pin (24) defining pivot axis (S) at which the first branch (11) is pivotably supported,
having an electric connection device (29) for an external electric connection (30), wherein the electric connection device (29) is electrically connected with a first tissue contact surface (17) of the first branch (11) via a first conductor (31) and is electrically connected with a second tissue contact surface (18) of the second branch (12) via a second conductor (32),
wherein the second conductor (32) is integrally formed and comprises a contact section (44) with at least one contact part (45) at its distal end (43), wherein the at least one contact part (45) abuts against an outer surface of an electrical conductive area of the support pin (24), such that an electrical rotation connection is established,
**characterized in that** the contact section (44) comprises at least one holding part (48) elastically biased toward the support pin (24), wherein one contact part (45) or multiple contact parts (45) of the at least one contact part (45) are arranged at each holding part (48).

2. Electrosurgical instrument according to claim 1, wherein the electric connection device (29) is arranged at the first branch (11).

3. Electrosurgical instrument according to claim 1 or 2, wherein an electrically insulating intermediate layer (55) is arranged between the first conductor (31) and the second conductor (32).

4. Electrosurgical instrument according to any of the preceding claims, wherein each branch (11, 12) has an electrically conductive core (33) and an electrically insulating outer layer (34) that encloses the core (33) at least partly.

5. Electrosurgical instrument according to claim 4, wherein an electrically insulating intermediate layer (55) is arranged between the second conductor (32) and the core (33) of the first branch (11).

6. Electrosurgical instrument according to claim 3 or 5, wherein the electrically insulating intermediate layer (55) is formed by a layer that is fixedly applied on the second conductor (32).

7. Electrosurgical instrument according to any of the preceding claims, wherein the second conductor (32) comprises a conductor section (42) having a rectangular cross-section and adjoining the contact section (44).

8. Electrosurgical instrument according to claim 7, wherein the second conductor (32) comprises a connection section (41) adjoining the conductor section (42), wherein the conductor section (42) is electrically connected with the connection device (29).

9. Electrosurgical instrument according to any of the preceding claims, wherein the contact section (44) completely surrounds the support pin (24) in a circumferential direction about the pivot axis (S).

10. Electrosurgical instrument according to any of the preceding claims, wherein the contact section (44) comprises at least one support part (47) by means of which the contact section (44) is supported at the first branch (11) on a radially outer side opposite the support pin (24).

11. Electrosurgical instrument according to claim 10, wherein a through hole (49) is provided between the at least one support part (47) and the at least one contact part (45) that completely extends through the contact section (44).

12. Electrosurgical instrument according to any of the preceding claims, wherein the support pin (24) comprises a conical section against which the at least one contact part (45) of the contact section (44) abuts.

13. Electrosurgical instrument according to claim 12, wherein a pressing part (51) is provided that creates an axial force parallel to the pivot axis (S) on the at least one contact part (45).

14. Method for manufacturing of an electrosurgical instrument (10) according to any of the claims 1 to 13 comprising the following steps:
- Separating a first conductor (31) from a plate (39) and separating a second conductor (32) from a plate (39),
- Electrically connecting the first conductor (31) with a first tissue contact surface (17),
- Arranging of the first tissue contact surface (17), the first conductor (31) and the second conductor (32) in a first die and manufacturing a first branch (11) by inserting a pourable, curable material in the first die, such that the at least one contact part (45) of a contact section (44) of the second conductor (32) is arranged in a support cavity (25) of the first branch (11),
- Arranging of an electrically conductive support pin (24) and a second tissue contact surface (18) electrically connected with the support pin (24) in a second die and manufacturing a second branch (12) by inserting a pourable, curable material in the second die,
- Inserting the support pin (24) of the second branch (12) in the support cavity (25) of the first branch (11) for arranging the two branches (11, 12) in a hinged manner at each other.

## Revendications

1. Instrument électrochirurgical (10),
comprenant une première branche (11) et une deuxième branche (12) dotée d'un tourillon (24) qui définit un axe de pivotement (S) et sur lequel la première branche (11) est montée avec possibilité de pivotement,
comprenant un dispositif de connexion électrique (29) pour un connecteur électrique (30) externe, le dispositif de connexion électrique (29) étant relié électriquement via un premier conducteur (31) à une première surface de contact de tissu (17) de la première branche (11), et étant relié électriquement via un deuxième conducteur (32) à une deuxième surface de contact de tissu (18) de la deuxième branche (12),
le deuxième conducteur (32) étant réalisé d'une seule pièce et présentant à son extrémité distale (43) une partie de contact (44) comportant au moins un élément de contact (45) qui est appliqué contre une surface extérieure d'une zone électriquement conductrice du tourillon (24), de manière à établir une connexion électrique rotative,
**caractérisé en ce que** la partie de contact (44) présente au moins un élément de support (48) qui est mis sous précontrainte élastique en direction du tourillon (24) et sur lequel sont disposés respectivement un élément de contact (45) ou plusieurs éléments de contact (45) parmi l'élément de contact (45), au nombre d'au moins un.

2. Instrument électrochirurgical selon la revendication 1, dans lequel le dispositif de connexion électrique (29) est placé sur la première branche (11).

3. Instrument électrochirurgical selon la revendication 1 ou 2, dans lequel une couche intermédiaire (55) électriquement isolante est disposée entre le premier conducteur (31) et le deuxième conducteur (32).

4. Instrument électrochirurgical selon l'une des revendications précédentes, dans lequel chaque branche (11, 12) présente un coeur (33) électriquement conducteur et une couche extérieure (34) électriquement isolante qui entoure au moins partiellement le coeur (33).

5. Instrument électrochirurgical selon la revendication 4, dans lequel une couche intermédiaire (55) électriquement isolante est disposée entre le deuxième conducteur (32) et le coeur (33) de la première branche (11).

6. Instrument électrochirurgical selon la revendication 3 ou 5, dans lequel la couche intermédiaire (55) électriquement isolante est constituée d'une couche appliquée solidement sur le deuxième conducteur (32).

7. Instrument électrochirurgical selon l'une des revendications précédentes, dans lequel le deuxième conducteur (32) présente une partie de conducteur (42) de section transversale rectangulaire, qui se raccorde à la partie de contact (44).

8. Instrument électrochirurgical selon la revendication 7, dans lequel le deuxième conducteur (32) présente une partie de raccordement (41) qui se raccorde à la partie de conducteur (42) et est reliée électriquement au dispositif de connexion (29).

9. Instrument électrochirurgical selon l'une des revendications précédentes, dans lequel la partie de contact (44) entoure complètement le tourillon (24) dans une direction périphérique autour de l'axe de pivotement (S).

10. Instrument électrochirurgical selon l'une des revendications précédentes, dans lequel la partie de contact (44) présente au moins un élément d'appui (47) au moyen duquel la partie de contact (44) prend appui sur la première branche (11), sur un côté radialement extérieur opposé au tourillon (24).

11. Instrument électrochirurgical selon la revendication 10, dans lequel un perçage (49) traversant complètement la partie de contact (44) est prévu entre l'élément d'appui (47), au nombre d'au moins un, et l'élément de contact (45), au nombre d'au moins un.

12. Instrument électrochirurgical selon l'une des revendications précédentes, dans lequel le tourillon (24) présente une partie conique contre laquelle est appliqué l'élément de contact (45), au nombre d'au moins un, de la partie de contact (44).

13. Instrument électrochirurgical selon la revendication 12, dans lequel il est prévu un élément de pression (51) qui exerce une force axiale sur l'élément de contact (45), au nombre d'au moins un, parallèlement à l'axe de pivotement (S).

14. Procédé de fabrication d'un instrument électrochirurgical (10) selon l'une des revendications 1 à 13, comprenant les étapes suivantes :
- séparation d'un premier conducteur (31) à partir d'une plaque (39), et d'un deuxième conducteur (32) à partir d'une plaque (39),
- raccordement électrique du premier conducteur (31) à une première surface de contact de tissu (17),
- mise en place de la première surface de contact de tissu (17), du premier conducteur (31) et du deuxième conducteur (32) dans un premier moule, et fabrication d'une première branche (11) par introduction d'un matériau fluide durcissable dans le premier moule, de manière à ce qu'au moins un élément de contact (45) d'une partie de contact (44) du deuxième conducteur (32) soit disposé dans un évidement de palier (25) de la première branche (11),
- mise en place d'un tourillon (24) électriquement conducteur et d'une deuxième surface de contact de tissu (18), reliée électriquement au coeur (33), dans un deuxième moule, et fabrication d'une deuxième branche (12) par introduction d'un matériau fluide durcissable dans le deuxième moule,
- insertion du tourillon (24) de la deuxième branche (12) dans l'évidement de palier (25) de la première branche (11), en vue de l'assemblage articulé des deux branches (11, 12) l'une avec l'autre.
